Europäisches Patentamt

European Patent Office

Office européen des brevets

(19)

(11) Numéro de publication : **0 135 411**
**B1**

(12)                     **FASCICULE DE BREVET EUROPÉEN**

(45) Date de publication du fascicule du brevet :
02.12.87

(21) Numéro de dépôt : **84401481.1**

(22) Date de dépôt : **12.07.84**

(51) Int. Cl.⁴ : **C 07 C 45/30**, C 07 C 45/64,
C 07 C 49/713,
C 07 C 49/687, C 07 C 45/80

(54) Procédé de préparation de l'hydroxy-4 triméthyl-2,4,6 cyclohexadiène-2,5 one.

(30) Priorité : **13.07.83 FR 8311708**

(43) Date de publication de la demande :
**27.03.85 Bulletin 85/13**

(45) Mention de la délivrance du brevet :
**02.12.87 Bulletin 87/49**

(84) Etats contractants désignés :
**AT BE CH DE FR GB IT LI LU NL SE**

(56) Documents cités :
DE-A- 2 019 196
JP-A-58 116 435
ACTA CHEMICA SCANDINAVICA, vol. B36, no. 10,
1982, pages 675-683; K.-E. BERGQUIST et al.: "Electrophilic chlorination of 4-methylphenols with molecular chlorine. Synthesis of dimethoxy aromatics by
methanolysis of 4-chloro-4-methylcyclohexa-2,5-die-
nones"
Le dossier contient des informations techniques
présentées postérieurement au dépôt de la demande
et ne figurant pas dans le présent fascicule.

(73) Titulaire : **RHONE-POULENC SANTE**
**Les Miroirs 18 Avenue d'Alsace**
**F-92400 Courbevoie Cédex (FR)**

(72) Inventeur : **Costantini, Michel**
**9 place Aristide Briand**
**F-69003 Lyon (FR)**
Inventeur : **Igersheim, Françoise**
**21 rue Florian**
**F-69100 Villeurbanne (FR)**
Inventeur : **Krumenacker, Léon**
**2 allée du Bois-Rond**
**F-69360 Serezin-du-Rhône (FR)**

(74) Mandataire : **Pilard, Jacques et al**
**RHONE-POULENC RECHERCHES .Service Brevets**
**Pharma 25, Quai Paul Doumer**
**F-92408 Courbevoie Cedex (FR)**

EP 0 135 411 B1

# 0 135 411

## Description

La présente invention concerne un nouveau procédé de préparation de l'hydroxy-4 triméthyl-2,4,6 cyclohexadiène-2,5 one qui constitue un intermédiaire pour la synthèse de la triméthylhydroquinone (TMHQ) qui est elle-même un précurseur de la vitamine E.

Il est connu de préparer l'hydroxy-4 triméthyl-2,4,6 cyclohexadiène-2,5 one par oxydation du triméthyl-2,4,6 phénol au moyen par exemple d'un peracide ou d'oxygène moléculaire en milieu basique. Cependant l'oxydation à l'air sous une pression voisine de 100 bars présente des difficultés de réalisation technique liées à des problèmes importants de sécurité.

Une hydroxy-4 cyclohexadiène-2,5 one peut aussi être obtenue par solvolyse d'une chloro-4 cyclohexadiène-2,5 one en présence d'eau et d'un sel d'argent selon le procédé décrit par A. Nilsson et coll., Tetrahedron Letters, 1107 (1975), ou bien, dans le cas particulier de l'hydroxy-4 tri-t.butyl-2,4,6 cyclohexadiène-2,5 one, par action de la soude aqueuse à température élevée sur la chloro-4 tri-t.butyl-2,4,6 cyclohexadiène-2,5 one selon le procédé décrit dans la demande de brevet allemand DE 2 019 196.

Une chloro-4 cyclohexadiène-2,5 one peut être obtenue par action du chlore gazeux dans un solvant organique tel que le dichlorométhane ou le diméthylformamide sur un alcoyl-4 phénol selon le procédé décrit par K. E. Bergquist et coll., Acta Chimica Scandinavia, B 36, 675 (1982). Cependant dans le cas particulier du triméthyl-2,4,6 phénol il se forme un mélange de chloro-4 triméthyl-2,4,6 cyclohexadiène-2,5 one et de chloro-3 triméthyl-2,4,6 phénol. Toutefois des rendements quantitatifs en chloro-4 triméthyl-2,4,6 cyclohexadiène-2,5 one peuvent être obtenus en effectuant la chloration dans l'anhydride acétique à une température de — 78 °C selon le procédé décrit par A. Fischer et G. N. Henderson, Can. J. Chem., 57, 552 (1979).

L'action du chlore sur le triméthyl-2,4,6 phénol ou bien conduit à un mélange de chloro-4 triméthyl-2,4,6 cyclohexadiène-2,5 one et de chloro-3 triméthyl-2,4,6 phénol ou bien nécessite, pour obtenir de bons rendements, l'emploi d'un solvant coûteux tel que l'anhydride acétique.

Par ailleurs, la transformation connue de la chloro-4 triméthyl-2,4,6 cyclohexadiène-2,5 one en hydroxy-4 triméthyl-2,4,6 cyclohexadiène-2,5 one nécessite l'emploi d'un sel d'argent ce qui rend le procédé difficilement utilisable industriellement.

D'après la demande de brevet européen EP 84 158, l'hydroxy-4 triméthyl-2,4,6 cyclohexadiène-2,5 one peut être obtenue par oxydation du triméthyl-2,4,6 phénol par un hypochlorite alcalin.

Il a maintenant été trouvé, et c'est ce qui fait l'objet de la présente invention, que l'on peut améliorer considérablement la préparation de l'hydroxy-4 triméthyl-2,4,6 cyclohexadiène-2,5 one en faisant réagir sur le triméthyl-2,4,6 phénol un agent d'halogénation et l'eau dans un solvant organique inerte vis-à-vis de l'agent d'halogénation.

Selon la présente invention, l'hydroxy-4 triméthyl-2,4,6 cyclohexadiène-2,5 one peut être obtenue :
— soit en faisant réagir le chlore dans un solvant organique inerte choisi parmi les hydrocarbures aliphatiques ou cycloaliphatiques éventuellement halogénés, les éthers et les esters d'acides carboxyliques en présence d'une base minérale choisie parmi les hydroxydes et les bicarbonates de métaux alcalins, puis avec l'eau, en présence ou non d'une base minérale, à un pH compris entre 0 et 10,
— soit en faisant réagir le triméthyl-2,4,6 phénol avec le chlore et l'eau dans un solvant organique inerte choisi parmi les hydrocarbures aliphatiques ou cycloaliphatiques éventuellement halogénés, les éthers et les esters d'acides carboxyliques en présence d'une base minérale choisie parmi les hydroxydes et bicarbonates de métaux alcalins à un pH compris entre 0 et 10.

Selon les conditions utilisées, la transformation du triméthyl-2,4,6 phénol en hydroxy-4 triméthyl-2,4,6 cyclohexadiène-2,5 one peut donc être réalisée en une seule étape, l'halogénation et l'hydrolyse s'effectuant simultanément, ou bien en deux étapes le produit d'halogénation étant ensuite hydrolysé.

Lorsque le procédé est mis en œuvre en deux étapes, l'halogénation peut être réalisée en milieu anhydre.

Comme agents d'halogénation, conviennent particulièrement bien les halogènes moléculaires (chlore), le chlorure de sulfuryle ou tout autre moyen capable de générer du chlore in situ.

Généralement on utilise un rapport molaire agent d'halogénation/triméthyl-2,4,6 phénol compris entre 0,5 et 2, de préférence entre 0,8 et 1,2.

Comme solvants, peuvent être utilisés les hydrocarbures aliphatiques ou cycloaliphatiques éventuellement halogénés (hexane, méthylcyclohexane, tétrachlorure de carbone, chloroforme, chlorure de méthylène, dichloro et trichloroéthane, solvants chlorés et fluorés), les éthers (méthylbutyléthers, dioxanne, tétrahydrofuranne), les acides carboxyliques aliphatiques (acide acétique), des esters organiques d'acides aliphatiques ou de l'acide phosphorique (acétate d'éthyle, triéthylphosphate, tributylphosphate), des amides (diméthylformamide, N-méthylpyrrolidone) ou leurs mélanges.

Généralement l'halogénation est effectuée à une température comprise entre 0 °C et la température d'ébullition du mélange réactionnel.

La concentration du triméthyl-2,4,6 phénol dans le solvant est généralement comprise entre 1 % et le taux de saturation du triméthyl-2,4,6 phénol dans le solvant considéré. De préférence, la concentration est comprise entre 5 et 15 % (poids/volume).

La phase d'halogénation peut être réalisée dans un éther ou un amide, éventuellement en association

2

avec un hydrocarbure aliphatique ou cycloaliphatique éventuellement halogéné, un acide carboxylique ou un ester d'acide carboxylique aliphatique ou d'acide phosphorique, ou bien dans un hydrocarbure aliphatique ou cycloaliphatique éventuellement halogéné, un éther, un acide carboxylique aliphatique, un ester d'acide carboxylique aliphatique ou d'acide phosphorique ou un amide en présence d'une base minérale ou organique.

Généralement, on utilise une base minérale ou organique soluble ou insoluble dans le solvant choisi. Le rapport moléculaire entre l'agent basique et l'agent d'halogénation est compris entre 1 et 10 et de préférence entre 1 et 2. Cependant l'halogénation se produit avec des résultats satisfaisants lorsque l'on utilise une mole d'agent basique par mole d'agent d'halogénation.

Les bases qui conviennent particulièrement bien sont choisies parmi les bases minérales (soude, potasse, lithine, bicarbonate et carbonate de sodium ou de potassium, chaux, carbonate de calcium, acétate de sodium ou de potassium) ou organique (amines, pyridine, pyridines substituées).

De préférence, on effectue la chloration en faisant barboter le chlore gazeux dans une solution agitée de triméthyl-2,4,6 phénol dans le solvant organique éventuellement en présence d'une base minérale ou organique, un excès de base par rapport au triméthyl-2,4,6 phénol mis en jeu pouvant être utilisé.

La chloration est généralement réalisée à une température comprise entre 0 °C et la température d'ébullition du mélange réactionnel.

Quelle que soit la méthode d'halogénation utilisée, l'hydroxy-4 triméthyl-2,4,6 cyclohexadiène-2,5 one est obtenue après traitement du produit d'halogénation par l'eau en opérant à un pH compris entre 10 et 0 et de préférence entre 9 et 0. Le pH du milieu réactionnel peut éventuellement être contrôlé par addition d'une base.

Il est possible d'ajouter l'eau au mélange réactionnel après élimination éventuelle de la base insoluble par filtration. Cependant on peut aussi isoler le produit d'halogénation du mélange réactionnel après filtration éventuelle et évaporation du solvant, et éventuellement la purifier. Dans l'un ou l'autre cas, l'hydrolyse est réalisée en agitant le produit brut ainsi obtenu en suspension dans l'eau, éventuellement en présence d'une base.

Quel que soit le produit sur lequel s'effectue l'hydrolyse, produit dans le milieu réactionnel, produit brut ou produit purifié, l'hydrolyse s'effectue par l'eau à une température comprise généralement entre 0 °C et la température d'ébullition du mélange réactionnel.

L'hydrolyse peut être effectuée en milieu hétérogène lorsque le solvant et l'eau sont peu miscibles ou en phase homogène lorsque le solvant et l'eau sont miscibles ou lorsqu'un solvant destiné à homogénéiser le milieu réactionnel est ajouté au mélange de solvant et d'eau non miscible.

Lorsque le procédé selon l'invention est mis en œuvre en une seule étape, la transformation du triméthyl-2,4,6 phénol en hydroxy-4 triméthyl-2,4,6 cyclohexadiène-2,5 one peut être réalisée en milieu homogène ou hétérogène.

L'halogénation et l'hydrolyse simultanées sont réalisées en traitant par un agent d'halogénation le triméthyl-2,4,6 phénol en solution dans un solvant organique en présence d'une quantité suffisante d'eau, c'est-à-dire au moins une mole d'eau par mole de triméthyl-2,4,6 phénol mis en œuvre, éventuellement en présence d'une base minérale ou organique de façon que le pH du mélange réactionnel soit compris entre 10 et 0 et de préférence entre 9 et 0.

Comme agents d'halogénation peuvent être utilisés ceux qui sont généralement utilisés pour l'halogénation en milieu anhydre, dans les mêmes rapports molaires que ceux indiqués précédemment.

En opérant selon l'une ou l'autre des variantes du procédé selon l'invention, le taux de transformation du triméthyl-2,4,6 phénol est généralement supérieur à 80 % et le rendement en hydroxy-4 triméthyl-2,4,6 cyclohexadiène-2,5 one est généralement supérieur à 60 %.

Un autre objet de l'invention concerne le procédé d'isolement de l'hydroxy-4 triméthyl-2,4,6 cyclohexadiène-2,5 one. Lorsque l'on opère en présence d'un solvant non miscible à l'eau, l'hydroxy-4 triméthyl-2,4,6 cyclohexadiène-2,5 one peut être isolée par extraction de la phase organique contenant l'hydroxy-4 triméthyl-2,4,6 cyclohexadiène-2,5 one par l'eau suivie de l'évaporation de la phase aqueuse ou de l'extraction de la solution aqueuse par un solvant organique non miscible qu'il suffit ensuite d'évaporer.

L'hydroxy-4 triméthyl-2,4,6 cyclohexadiène-2,5 one peut être transformée en triméthylhydroquinone dans les conditions décrites dans le brevet français 7 333 374 publié sous le numéro 2 200 225 c'est-à-dire par chauffage à une température d'au moins 100 °C dans un milieu liquide non acide tel que le méthanol et un milieu aqueux.

Les exemples suivants, donnés à titre non limitatif, montrent comment l'invention peut être mise en pratique.

## Exemple 1

Dans un réacteur cylindrique de 250 cm³ comportant une agitation centrale, un tube plongeant d'arrivée de chlore, un réfrigérant ascendant, un thermomètre et un système de régulation thermique, on charge :

triméthyl-2,4,6 phénol                                        10,9 g (80 mMoles)

| | |
|---|---|
| tétrachlorure de carbone | 160 cm$^3$ |
| bicarbonate de sodium | 10,55 g (120,8 mMoles) |

On agite à une vitesse de 1 000 tours/minute et on maintient le mélange réactionnel à 0 °C tout en faisant passer un courant de chlore gazeux au débit de 5 litres/heure pendant 21 minutes (soit 78,1 mMoles de chlore).

Après filtration, le mélange réactionnel est versé dans un réacteur agité contenant 1 litre d'eau et 6,7 g de bicarbonate de sodium soit 80 mMoles. Après 2 heures d'agitation à une température voisine de 20 °C, le mélange réactionnel est décanté et la phase aqueuse est extraite par 2 fois 500 cm$^3$ d'éther isopropylique. Les phases organiques obtenues sont réunies. On dose, par chromatographie gaz-liquide, le triméthyl-2,4,6 phénol résiduel, l'hydroxy-4 triméthyl-2,4,6 cyclohexadiène-2,5 one et le chloro-3 triméthyl-2,4,6 phénol formés. Les résultats suivants sont obtenus :
— taux de transformation du triméthyl-2,4,6 phénol : 88,5 %
— rendement molaire en hydroxy-4 triméthyl-2,4,6 cyclohexadiène-2,5 one par rapport au triméthyl-2,4,6 phénol transformé : 70 %
— rendement en chloro-3 triméthyl-2,4,6 phénol : 10 %.

A titre de comparaison, en opérant dans les conditions décrites ci-dessus mais en absence de bicarbonate de sodium, on obtient les résultats suivants :
— taux de transformation du triméthyl-2,4,6 phénol : 84 %
— rendement en chloro-3 triméthyl-2,4,6 phénol : voisin de 80 %
— rendement en hydroxy-4 triméthyl-2,4,6 cyclohexadiène-2,5 one : traces.

## Exemple 2

On opère comme dans l'exemple 1 en remplaçant le bicarbonate de sodium par du diméthylformamide (98 mMoles). On obtient les résultats suivants :
— taux de transformation du triméthyl-2,4,6 phénol : 74,5 %
— rendement en hydroxy-4 triméthyl-2,4,6 cyclohexadiène-2,5 one : 62 %.

## Exemple 3

On opère comme dans l'exemple 1 en remplaçant le bicarbonate de sodium par de la pyridine (82 mMoles). On obtient les résultats suivants :
— taux de transformation du triméthyl-2,4,6 phénol : 88 %
— rendement en hydroxy-4 triméthyl-2,4,6 cyclohexadiène-2,5 one : 38 %.

## Exemple 4

On opère comme dans l'exemple 1 en remplaçant le bicarbonate de sodium par de la triéthylamine (82 mMoles). On obtient les résultats suivants :
— taux de transformation du triméthyl-2,4,6 phénol : 74 %
— rendement en hydroxy-4 triméthyl-2,4,6 cyclohexadiène-2,5 one : 49 %.

## Exemple 5

On opère comme dans l'exemple 1 en remplaçant le tétrachlorure de carbone par le chlorure de méthylène et en présence de 121 mMoles de bicarbonate de sodium. On obtient les résultats suivants :
— taux de transformation du triméthyl-2,4,6 phénol : 80,5 %
— rendement en hydroxy-4 triméthyl-2,4,6 cyclohexadiène-2,5 one : 68 %.

## Exemple 6

On opère comme dans l'exemple 1 en remplaçant le tétrachlorure de carbone par le diméthylformamide sans ajouter de bicarbonate de sodium. On obtient les résultats suivants :
— taux de transformation du triméthyl-2,4,6 phénol : 80,5 %
— rendement en hydroxy-4 triméthyl-2,4,6 cyclohexadiène-2,5 one : 50,5 %.

## Exemple 7

On opère comme dans l'exemple 1, en utilisant les quantités suivantes de produits :

| | |
|---|---|
| triméthyl-2,4,6 phénol | 21,8 g (160 mMoles) |
| tétrachlorure de carbone | 160 cm$^3$ |
| bicarbonate de sodium | 21,1 g (241,6 mMoles) |

On fait passer le courant de chlore gazeux au débit de 5 litres/heure pendant 42 minutes (soit 156,2 mMoles de chlore) dans le mélange réactionnel maintenu à 0 °C et agité à la vitesse de 1 000 tours/minute.

Après filtration le mélange réactionnel est versé dans un réacteur agité contenant 2 litres d'eau et 13,4 g de bicarbonate de sodium soit 160 mMoles. Après 2 heures d'agitation à une température voisine de 20 °C, le mélange réactionnel est traité dans les conditions de l'exemple 1. On obtient les résultats suivants :
— taux de transformation du triméthyl-2,4,6 phénol : 81,5 %
— rendement en hydroxy-4 triméthyl-2,4,6 cyclohexadiène-2,5 one : 59 %.

### Exemple 8

On opère comme dans l'exemple 1 mais en utilisant seulement 70,4 mMoles de chlore. On obtient les résultats suivants :
— taux de transformation du triméthyl-2,4,6 phénol : 78,5 %
— rendement en hydroxy-4 triméthyl-2,4,6 cyclohexadiène-2,5 one : 69,5 %.

### Exemple 9

Dans un réacteur cylindrique comportant une agitation centrale, un tube plongeant d'arrivée de chlore, un réfrigérant ascendant, un thermomètre et un système de régulation thermique, on charge :

| | |
|---|---|
| triméthyl-2,4,6 phénol | 13,6 g (100 mMoles) |
| tétrachlorure de carbone | 200 cm$^3$ |
| bicarbonate de sodium | 16,8 g (200 mMoles) |

On agite à une vitesse de 1 000 tours/minute. Le mélange réactionnel étant maintenu à 0 °C, on fait passer un courant de chlore gazeux au débit de 5 litres/heure pendant 26 minutes (soit 96,7 mMoles de chlore).

On laisse la température remonter à 20 °C puis on ajoute rapidement 200 cm$^3$ d'eau. On poursuit ensuite l'agitation pendant 2 heures. Après décantation, la couche aqueuse est extraite par 4 fois 50 cm$^3$ d'éther isopropylique.

Dans la phase contenant le tétrachlorure de carbone, on dose, par chromatographie gaz-liquide :
— triméthyl-2,4,6 phénol : 1,78 g (13,06 mMoles)
— hydroxy-4 triméthyl-2,4,6 cyclohexadiène-2,5 one : 8,44 g (55,5 mMoles).

Dans la phase éthérée, on dose, par chromatographie gaz-liquide :
— triméthyl-2,4,6 phénol : 0 %
— hydroxy-4 triméthyl-2,4,6 cyclohexadiène-2,5 one : 1,61 g (10,6 mMoles).

Le bilan global est le suivant :
— taux de transformation du triméthyl-2,4,6 phénol : 86,9 %
— rendement en hydroxy-4 triméthyl-2,4,6 cyclohexadiène-2,5 one : 76 %.

### Exemple 10

On opère dans les mêmes conditions que dans l'exemple 9 en utilisant les charges suivantes :

| | |
|---|---|
| triméthyl-2,4,6 phénol | 13,6 g (0,1 mole) |
| tétrachlorure de carbone | 200 cm$^3$ |
| bicarbonate de sodium | 16,8 g (0,2 mole). |

Dans le mélange réactionnel agité et maintenu à 25 °C, on fait passer 0,098 mole de chlore gazeux. On ajoute ensuite 200 cm$^3$ d'eau puis on agite le mélange réactionnel pendant 2 heures 30 à 25 °C. La phase aqueuse, après séparation par décantation, est extraite par de l'éther isopropylique. Les phases organiques sont réunies. L'analyse de la phase organique par chromatographie gaz-liquide, montre que :
— le taux de transformation du triméthyl-2,4,6 phénol est de 88 %
— le rendement en hydroxy-4 triméthyl-2,4,6 cyclohexadiène-2,5 one est de 71,5 %.

### Exemple 11

On opère comme dans l'exemple 1, en utilisant les produits suivants :

| | |
|---|---|
| triméthyl-2,4,6 phénol | 10,9 g (80 mMoles) |
| tétrachlorure de carbone | 160 cm$^3$ |
| bicarbonate de sodium | 10,55 g (120,8 mMoles). |

Dans le mélange réactionnel agité et maintenu à 0 °C, on fait passer 78 mMoles de chlore gazeux en 21 minutes. Après filtration, le mélange réactionnel est versé dans un réacteur contenant 1 000 cm³ d'un mélange eau-tétrahydrofuranne (20-80 en volume) et 9,2 g de bicarbonate de sodium. Le mélange homogène est agité pendant 2 heures à une température voisine de 25 °C. Le tétrachlorure de carbone et le tétrahydrofuranne sont éliminés par distillation sous pression réduite puis la phase aqueuse est extraite par l'éther isopropylique. L'analyse de la phase organique montre que :
— le taux de transformation du triméthyl-2,4,6 phénol est de 84 %
— le rendement en hydroxy-4 triméthyl-2,4,6 cyclohexadiène-2,5 one est de 66 %.

## Exemple 12

On opère dans le même appareillage que celui utilisé dans l'exemple 9 en utilisant les charges suivantes :

| | |
|---|---|
| triméthyl-2,4,6 phénol | 13,6 g (0,1 mole) |
| tétrachlorure de carbone | 200 cm³ |
| carbonate de sodium | 10,6 g (0,1 mole) |

Dans le milieu réactionnel agité et maintenu à 0 °C, on fait passer 0,098 mole de chlore gazeux puis on laisse remonter la température au voisinage de 20 °C. On ajoute alors 200 cm³ d'eau puis on agite le mélange réactionnel pendant 2 heures 30 à une température voisine de 20 °C. Après séparation par décantation, la phase aqueuse est extraite par l'éther isopropylique. Les phases organiques sont réunies. L'analyse par chromatographie gaz-liquide de la phase organique montre que :
— le taux de transformation du triméthyl-2,4,6 phénol est de 83 %
— le rendement en hydroxy-4 triméthyl-2,4,6 cyclohexadiène-2,5 one est de 77,5 %.

## Exemple 13

On opère comme dans l'exemple 12 en remplaçant 0,1 mole de carbonate de sodium par 0,1 mole de soude. On obtient les résultats suivants :
— taux de transformation du triméthyl-2,4,6 phénol : 81,3 %
— rendement en hydroxy-4 triméthyl-2,4,6 cyclohexadiène-2,5 one : 65 %.

## Exemple 14

On opère dans le même appareillage que celui utilisé dans l'exemple 9 en utilisant les charges suivantes :

| | |
|---|---|
| triméthyl-2,4,6 phénol | 13,6 g (0,1 mole) |
| tétrachlorure de carbone | 200 cm³ |
| bicarbonate de sodium | 8,4 g (0,1 mole) |

Dans le mélange réactionnel agité et maintenu à 0 °C, on fait passer 0,098 mole de chlore gazeux. Après retour du mélange réactionnel à une température voisine de 20 °C, on ajoute 200 cm³ d'eau puis agite pendant 2 heures 30.
La phase aqueuse est séparée par décantation puis est extraite par de l'éther isopropylique. Les phases organiques sont réunies. L'analyse de la phase organique par chromatographie gaz-liquide montre que :
— le taux de transformation du triméthyl-2,4,6 phénol est de 75,9 %
— le rendement en hydroxy-4 triméthyl-2,4,6 cyclohexadiène-2,5 one est de 72,5 %.

## Exemple 15

On opère dans le même appareillage que celui utilisé dans l'exemple 9. On introduit :

| | |
|---|---|
| tétrachlorure de carbone | 200 cm³ |
| eau | 200 cm³ |
| triméthyl-2,4,6 phénol | 13,6 g (0,1 mole) |
| bicarbonate de sodium | 16,8 g (0,2 mole) |

Le pH du mélange initial est égal à 8. Le mélange réactionnel est refroidi à 0 °C puis on fait passer 0,098 mole de chlore en 24 minutes. On laisse alors la température remonter au voisinage de 20 °C. L'agitation est poursuivie pendant 2 heures 30 à cette température. Après extraction dans les conditions habituelles, l'analyse de la phase organique par chromatographie gaz-liquide montre que :
— le taux de transformation du triméthyl-2,4,6 phénol est de 81,3 %

— le rendement en hydroxy-4 triméthyl-2,4,6 cyclohexadiène-2,5 one est de 77 %.

Exemple 16

On opère dans le même appareillage que celui utilisé dans l'exemple 9. On introduit :

| | |
|---|---|
| tétrachlorure de carbone | 200 cm$^3$ |
| eau | 200 cm$^3$ |
| triméthyl-2,4,6 phénol | 13,6 g (0,1 mole). |

Dans le mélange réactionnel refroidi à 0 °C et agité, on fait passer 0,098 mole de chlore en 24 minutes. On laisse la température remonter au voisinage de 20 °C puis on poursuit l'agitation pendant 2 heures 30. Après extraction dans les conditions habituelles, l'analyse de la phase organique par chromatographie gaz-liquide montre que :
— le taux de transformation du triméthyl-2,4,6 phénol est de 73,4 %
— le rendement en hydroxy-4 triméthyl-2,4,6 cyclohexadiène-2,5 one est de 66,8 %.

Exemple 17

On utilise une phase contenant le tétrachlorure de carbone provenant de la chloration du triméthyl-2,4,6 phénol dont l'analyse donne la composition suivante :

| | |
|---|---|
| tétrachlorure de carbone | 1 400  g |
| triméthyl-2,4,6 phénol | 11,5 g |
| hydroxy-4 triméthyl-2,4,6 cyclohexadiène-2,5 one | 33,5 g |

La phase contenant le tétrachlorure de carbone est extraite 4 fois avec 1 200 cm$^3$ d'eau distillée. La phase aqueuse ainsi obtenue est extraite 8 fois avec 600 cm$^3$ d'éther isopropylique. La phase éthérée ainsi obtenue est lavée 2 fois avec 50 cm$^3$ d'eau, séchée sur sulfate de sodium puis concentrée à sec. On obtient ainsi 24,2 g d'hydroxy-4 triméthyl-2,4,6 cyclohexadiène-2,5 one dont les caractéristiques sont les suivantes :
— point de fusion                                                  41 °C
— composition : hydroxy-4 triméthyl-2,4,6 cyclohexadiène-2,5 one : 95 %
                           triméthyl-2,4,6 phénol : 1 %

Exemple 18

Dans un autoclave en acier inoxydable, on introduit :

| | |
|---|---|
| eau | 200 cm$^3$ |
| hydroxy-4 triméthyl-2,4,6 cyclohexadiène-2,5 one (obtenu à l'exemple 17) | 4,48  g |
| soude | 0,056 g |
| sulfite de sodium | 0,2  g |

Le mélange réactionnel est chauffé à 200 °C pendant 10 minutes. Après refroidissement, la triméthylhydroquinone qui a précipité est séparée par filtration. Le dosage de la triméthylhydroquinone dans le précipité et dans le filtrat montre que l'on obtient 3,9 g de triméthylhydroquinone. Le rendement est de 91 % par rapport à l'hydroxy-4 triméthyl-2,4,6 cyclohexadiène-2,5 one mise en œuvre.

Exemple 19

On opère dans le même appareillage que celui utilisé dans l'exemple 9. On introduit :

| | |
|---|---|
| méthyltertiobutyléther | 200 cm$^3$ |
| triméthyl-2,4,6 phénol | 13,6 g (100 mMoles) |
| bicarbonate de sodium | 16,8 g (200 mMoles) |

On agite à une vitesse de 1 000 tours/minute. Le mélange réactionnel étant maintenu à 40 °C, on fait passer un courant régulier de chlore gazeux pendant 25 minutes. La quantité totale de chlore engagé est de 110 mMoles.
On ajoute ensuite rapidement 200 cm$^3$ d'eau et on poursuit l'agitation pendant 30 minutes à 40 °C. Après décantation, la couche aqueuse est extraite par 4 fois 50 cm$^3$ de chlorure de méthylène. Les phases organiques sont réunies. L'analyse par chromatographie gaz-liquide de la phase organique montre que :
— le taux de transformation du triméthyl-2,4,6 phénol est de 98 %
— le rendement en hydroxy-4 triméthyl-2,4,6 cyclohexadiène-2,5 one est de 85 %.

## Exemple 20

On opère dans le même appareillage que celui utilisé dans l'exemple 9. On introduit :

| | |
|---|---|
| méthyltertiobutyléther | 200 cm$^3$ |
| triméthyl-2,4,6 phénol | 13,6 g (100 mMoles) |
| bicarbonate de sodium | 16,8 g (200 mMoles) |
| eau | 200 cm$^3$ |

On agite à une vitesse de 1 000 tours/minute. Le mélange réactionnel étant maintenu à 25 °C, on fait passer un courant régulier de chlore gazeux pendant 24 minutes. La quantité de chlore engagé est de 110 mMoles.

On poursuit ensuite l'agitation pendant 30 minutes à 40 °C. Après décantation, la couche aqueuse est extraite par 4 fois 50 cm$^3$ de chlorure de méthylène. Les phases organiques sont réunies. L'analyse par chromatographie gaz-liquide de la phase organique montre que :
— le taux de transformation du triméthyl-2,4,6 phénol est de 99,6 %
— le rendement en hydroxy-4 triméthyl-2,4,6 cyclohexadiène-2,5 one est de 89,8 %.

## Exemple 21

On opère dans le même appareillage que celui utilisé dans l'exemple 9. On introduit :

| | |
|---|---|
| méthyltertiobutyléther | 200 cm$^3$ |
| triméthyl-2,4,6 phénol | 13,6 g (100 mMoles) |
| eau | 200 cm$^3$ |

On agite à une vitesse de 1 000 tours/minute. Le mélange réactionnel étant maintenu à 25 °C, on fait passer un courant régulier de chlore gazeux pendant 25 minutes. La quantité de chlore engagé est de 110 mMoles.

On poursuit ensuite l'agitation pendant 30 minutes à 40 °C. Après décantation, la couche aqueuse est extraite par 4 fois 50 cm$^3$ de chlorure de méthylène. Les phases organiques sont réunies. L'analyse par chromatographie gaz-liquide de la phase organique montre que :
— le taux de transformation du triméthyl-2,4,6 phénol est de 98,1 %
— le rendement en hydroxy-4 triméthyl-2,4,6 cyclohexadiène-2,5 one est de 90,8 %.

## Exemple 22

On opère dans le même appareillage que celui utilisé dans l'exemple 9. On introduit :

| | |
|---|---|
| méthyltertiobutyléther | 200 cm$^3$ |
| triméthyl-2,4,6 phénol | 13,6 g (100 mMoles) |

On agite à une vitesse de 1 000 tours/minute. Le mélange réactionnel étant maintenu à 20 °C, on fait passer un courant régulier de chlore gazeux pendant 30 minutes. La quantité de chlore engagé est de 110 mMoles. L'analyse montre que ce chlore a été intégralement consommé.

On ajoute ensuite rapidement 200 cm$^3$ d'eau contenant 16,8 g (200 mMoles) de bicarbonate de sodium puis on poursuit l'agitation pendant 30 minutes à 40 °C. Après décantation, la couche aqueuse est extraite par 4 fois 50 cm$^3$ de méthyltertiobutyléther. Les phases organiques sont réunies. L'analyse par chromatographie gaz-liquide de la phase organique montre que :
— le taux de transformation du triméthyl-2,4,6 phénol est de 80,4 %
— le rendement en hydroxy-4 triméthyl-2,4,6 cyclohexadiène-2,5 one est de 68,5 %.

## Exemple 23

On opère dans le même appareillage que celui utilisé dans l'exemple 9. On introduit :

| | |
|---|---|
| méthyltertiobutyléther | 150 cm$^3$ |
| triméthyl-2,4,6 phénol | 20,4 g (150 mMoles) |
| bicarbonate de sodium | 25,2 g (300 mMoles) |
| eau | 15 cm$^3$ |

On agite à une vitesse de 1 000 tours/minute. Le mélange réactionnel étant maintenu à 25 °C, on fait passer un courant régulier de chlore gazeux pendant 26 minutes. La quantité totale de chlore engagé est de 165 mMoles.

On ajoute ensuite rapidement 135 cm³ d'eau et on poursuit l'agitation pendant 30 minutes à 40 °C. Après décantation, la couche aqueuse est extraite par 4 fois 50 cm³ de méthyltertiobutyléther. Les phases organiques sont réunies. L'analyse par chromatographie gaz-liquide montre que :
— le taux de transformation du triméthyl-2,4,6 phénol est de 98,5 %
— le rendement en hydroxy-4 triméthyl-2,4,6 cyclohexadiène-2,5 one est de 84,5 %.

## Exemple 24

On opère dans le même appareillage que celui utilisé dans l'exemple 9. On introduit :

| | |
|---|---|
| méthyltertiobutyléther | 200 cm³ |
| triméthyl-2,4,6 phénol | 13,6 g (100 mMoles) |
| bicarbonate de sodium | 16,8 g (200 mMoles) |
| eau | 20 cm³ |

On agite à une vitesse de 1 000 tours/minute. Le mélange réactionnel étant maintenu à 50 °C, on fait passer un courant régulier de chlore gazeux pendant 28 minutes. La quantité totale de chlore engagé est de 110 mMoles.

On ajoute ensuite rapidement 180 cm³ d'eau et on poursuit l'agitation pendant 30 minutes à 40 °C. Après décantation, la couche aqueuse est extraite par 4 fois 50 cm³ de méthyltertiobutyléther. Les phases organiques sont réunies. L'analyse par chromatographie gaz-liquide montre que :
—·le taux de transformation du triméthyl-2,4,6 phénol est de 96 %
— le rendement en hydroxy-4 triméthyl-2,4,6 cyclohexadiène-2,5 one est de 85 %.

## Exemple 25

On opère dans le même appareillage que celui utilisé dans l'exemple 9. On introduit :

| | |
|---|---|
| acétate d'éthyle | 200 cm³ |
| triméthyl-2,4,6 phénol | 13,6 g (100 mMoles) |
| bicarbonate de sodium | 16,8 g (200 mMoles) |

On agite à une vitesse de 1 000 tours/minute. Le mélange réactionnel étant maintenu à 0 °C, on fait passer un courant régulier de chlore gazeux pendant 24 minutes. La quantité totale de chlore engagé est de 100 mMoles.

On ajoute ensuite rapidement 200 cm³ d'eau et on poursuit l'agitation pendant 3 heures à 25 °C. Après décantation, la couche aqueuse est extraite par 4 fois 50 cm³ de chlorure de méthylène. Les phases organiques sont réunies. L'analyse par chromatographie gaz-liquide de la phase organique montre que :
— le taux de transformation du triméthyl-2,4,6 phénol est de 81,5 %
— le rendement en hydroxy-4 triméthyl-2,4,6 cyclohexadiène-2,5 one est de 74 %.

## Exemple 26

On opère dans le même appareillage que celui utilisé dans l'exemple 9. On introduit :

| | |
|---|---|
| méthylcyclohexane | 200 cm³ |
| triméthyl-2,4,6 phénol | 13,6 g (100 mMoles) |
| bicarbonate de sodium | 16,8 g (200 mMoles) |

On agite à une vitesse de 1 000 tours/minute. Le mélange réactionnel étant maintenu à 25 °C, on fait passer un courant régulier de chlore gazeux pendant 26 minutes. La quantité totale de chlore engagé est de 98,5 mMoles.

On ajoute ensuite rapidement 200 cm³ d'eau et on poursuit l'agitation pendant 30 minutes à 25 °C. Après décantation, la couche aqueuse est extraite par 4 fois 50 cm³ de chlorure de méthylène. Les phases organiques sont réunies. L'analyse par chromatographie gaz-liquide de la phase organique montre que :
— le taux de transformation du triméthyl-2,4,6 phénol est de 95,5 %
— le rendement en hydroxy-4 triméthyl-2,4,6 cyclohexadiène-2,5 one est de 64,5 %.

## Revendications

1. Procédé pour la préparation d'hydroxy-4 triméthyl-2,4,6 cyclohexadiène-2,5 one caractérisé en ce que l'on fait réagir le triméthyl-2,4,6 phénol avec le chlore dans un solvant organique inerte choisi parmi les hydrocarbures aliphatiques ou cycloaliphatiques éventuellement halogénés, les éthers et les esters d'acides carboxyliques en présence d'une base minérale choisie parmi les hydroxydes et les bicarbonates de métaux alcalins, puis avec l'eau, en présence ou non d'une base minérale, à un pH compris entre 0 et 10.

2. Procédé de préparation d'hydroxy-4 triméthyl-2,4,6 cyclohexadiène-2,5 one caractérisé en ce que l'on fait réagir le triméthyl-2,4,6 phénol avec le chlore et l'eau dans un solvant organique inerte choisi parmi les hydrocarbures aliphatiques ou cycloaliphatiques éventuellement halogénés, les éthers et les esters d'acides carboxyliques en présence d'une base minérale choisie parmi les hydroxydes et bicarbonates de métaux alcalins à un pH compris entre 0 et 10.

**Claims**

1. Process for the preparation of 4-hydroxy-2,4,6-trimethyl-cyclohexa-2,5-dienone, characterized in that 2,4,6-trimethylphenol is reacted with chlorine in an inert organic solvent chosen from among aliphatic or cycloaliphatic, optionally halogenated, hydrocarbons, ethers and esters of carboxylic acids, in the presence of an inorganic base chosen from among the alkali metal hydroxides and bicarbonates, and thereafter with water in the presence or absence of an inorganic base, at a pH of between 0 and 10.

2. Process for the preparation of 4-hydroxy-2,4,6-trimethyl-cyclohexa-2,5-dienone, characterized in that 2,4,6-trimethylphenol is reacted with chlorine and water in an inert organic solvent chosen from among aliphatic or cycloaliphatic, optionally halogenated, hydrocarbons, ethers and esters of carboxylic acids, in the presence of an inorganic base chosen from among alkali metal hydroxides and bicarbonates, at a pH of between 0 and 10.

**Patentansprüche**

1. Verfahren zur Herstellung von 4-Hydroxy-2,4,6-trimethyl-2,5-cyclohexadien-on, dadurch gekennzeichnet, daß man 2,4,6-Trimethylphenol mit Chlor in einem inerten organischen Lösungsmittel, ausgewählt aus den gegebenenfalls halogenierten aliphatischen oder cycloaliphatischen Kohlenwasserstoffen, den Äthern und den Estern von Carbonsäuren in Gegenwart einer anorganischen Base, ausgewählt aus den Alkalimetallhydroxiden und -bicarbonaten, und dann mit Wasser in An- oder Abwesenheit einer anorganischen Base bei einem pH-Wert zwischen 0 und 10 reagieren läßt.

2. Verfahren zur Herstellung von 4-Hydroxy-2,4,6-trimethyl-2,5-cyclohexadien-on, dadurch gekennzeichnet, daß man das 2,4,6-Trimethylphenol mit dem Chlor und Wasser in einem inerten organischen Lösungsmittel, ausgewählt aus den gegebenenfalls halogenierten aliphatischen oder cycloaliphatischen Kohlenwasserstoffen, den Äthern und den Estern von Carbonsäuren in Gegenwart einer anorganischen Base, ausgewählt aus den Alkalimetallhydroxiden und -bicarbonaten, bei einem pH-Wert zwischen 0 und 10 reagieren läßt.